# EUROPEAN PATENT APPLICATION

(11) **EP 2 578 165 A1**
(43) Date of publication of application: **10.04.2013**
(21) Application number: 11008160.1
(22) Date of filing: 08.10.2011
(51) Int. Cl.: A61B 17/00, A61K 35/30

(54) **Method for stopping aging of the body by replacing of the old biocathode centers by young biocathode centers**

(71) Applicant: Denev, Martin Ivanov, 55445 Jönköping (SE)
(72) Inventor: Denev, Martin Ivanov, 55445 Jönköping (SE)

(57) **Abstract**

THE INVENTION BELONGS TO THE METHODS FOR MEDICAL TREATMENT OF THE BODY WITH AIM TO STOP AGING OF THE BODY AND TO CURE FROM CANCER.

MARTIN DENEV WAS DISCOVERING, THAT THE AGING OF THE BODY, IS CAUSED BY AGING OF THE BIOCATHODE CENTERS IN THE BODY AND THIS MAKE THE BODY CANCEROUS.

YOUNG BIOCATHODE CENTERS HAS RELATIVELY MORE INTENSIVE EMISSION OF ELECTRONS AND THIS RELATIVELY MORE INTENSIVE EMISSION OF ELECTRONS MAKE THE BODY DOMINANTLY ELECTRICALY NEGATIVE AND KEEPS THE BODY YOUNG AND IS HEALING THE BODY FROM CANCER, WHICH HAS POSITIVELY ELECTRICAL CHARGE.

OLD BIOCATHODE CENTERS HAS RELATIVELY LESS INTENSIVE EMISSION OF ELECTRONS AND THIS RELATIVELY LESS INTENSIVE EMISSION OF ELECTRONS MAKE THE BODY OLD AND CANCEROUS, BECAUSE THE CANCER IS ELECTRICALY POSITIVE.

BIOCATHODE CENTERS ARE MAKING THE BODY DOMINANTLY BASIC, AS THE BODIES OF THE CHILDREN.

THE BIOCATHODE CENTERS IN THE BODY ARE PLEXUS CHORIOIDEUS, EPIPHYSIS CEREBRY, GLOMUS CAROTICUM, GLOMUS SUPRACARDIALE AND GLOMUS COCCIGEUM.

THE DISCOVERY OF THE BIOCATHODE CENTERS WAS REGISTERED 22 OKTOBER 1964 AT THE BULGARIAN MINISTRY OF PUBLIC HEALTH AN SOCIAL CARE UNDER REGISTRATION NUMMBER X 1823 OF MARTIN DENEV.

THE PATENT CLAIM IS FOR METHOD FOR STOPPING AGING OF THE BODY BY REPLACING OF THE OLD BIOCATHODE CENTERS BY YOUNG BIOCATHODE CENTERS.

THE SHORT NAME OF THE BIOCATHODE CENTERS IS BCC.

## Description

METHOD FOR STOPPING AGING OF THE BODY, BY REPLACING OF THE OLD BIOCATHODE CENTERS IN THE BODY, BY YOUNG BIOCATHODE CENTERS.

THE INVENTION BELONGS TO THE AREA OF MEDICAL THERAPY METHODS.

MARTIN DENEV WAS DISCOVERING THAT PLEXUS CHORIOIDEUS, EPIPHYSIS CEREBRI, GLOMUS CAROTICUM, GLOMUS SUPRACARDIALE AND GLOMUS COCCYGEUM, ARE BIOCATHODE CENTERS IN THE BODY, WHICH, SIMILAR TO TECHNICAL CATHODES, ARE EMITTING ELECTRONS, WHICH ARE SUPPORTING THE HOMEOSTATIC BALANCE, BETWEEN THE NEGATIVELY AND POSITIVELY CHARGES IN THE BODY, BUT IN FAVOUR OF THE NEGATIVELY CHARGES.

MARTIN DENEV WAS DISCOVERING, THAT THE AGING OF THE BODY, IS CAUSED BY THE AGING OF THE BIOCATHODE CENTERS IN THE BODY.

THE AGING OF BIOCATHODE CENTERS IS THE PRIMARY PROCESS OF AGING AND THE AGING OF THE BODY IS SECONDARY PROCESS OF AGING.

MARTIN DENEV WAS DISCOVERING THAT, AS THE END OF THE CATHODE, IN A RADIO LAMP, IN A RADIO RECIEVER, MAKE THE RADIO LAMP OLD AND DEATH, THE END OF BIOCATHODE CENTERS IN THE BODY, MAKE THE BODY OLD AND DEATH.

THE REDUCED ELECTRON EMISSION OF THE BIOCATHODE CENTERS, MAKE THE BODY MORE SOUR, THAN BASIC.

CANCER IS ELECTRICALY POSITIVE AND CAN BE STOPPED BY \ELECTRONE EMISSION OF THE BIOCATHODE CENTERS, AS BY THE ANTIOXIDANTS, WHICH ARE ELECTRICALY NEGATIVE.

MARTIN DENEV WAS DISCOVERING, THAT, AS THE REPLACEMENT OF THE OLD CATHODE IN A RADIO LAMP, IN A RADIO RECEIVER OR CATHODE TV MONITOR, MAKE THE RADIO LAMPS AND CATHODE TV MONITORS YOUNG, THE REPLACEMENT OF THE OLD BIOCATHODES CENTERS IN THE BODY, BY YOUNG BIOCATHODE, CAN MAKE THE BODY YOUNG.

MARTIN DENEV HAD REGISTERED THE DISCOVERY OF THE BIOCATHODE CENTERS 22 OCTOBER 1964 AT THE BULGARIAN MINISTRY OF PUBLIC HEALTH AND SOCIAL CARE UNDER REGISTRATION NUMMBER X 1823. PRIORITY DOCUMENT NUMMBER 2 IS PROVING THIS.

MARTIN DENEV WAS SENDING FOR PUBLICATION THE DISCOVERY OF THE BIOCATHODE CENTERS TO BIOPHYSICAL JOURNAL TO THE INSTITUTE OF SCIENCE AND TECHNOLOGY OF THE UNIVERSITY OF MICHIGAN. THE BIOPHYSICAL JOURNAL WAS ACCEPTING THE ARTICLE, BUT MARTIN DENEV WAS PREVENTET BY THE BULGARIAN AUTHORITIES TO SUBMIT THE CLAIMED EXPERIMENTS AND THE ARTICLE WAS NOT PUBLISHED. PRIORITY DOCUMENT NUMMBER 2 IS PROVING THIS.

## Claims

1. METHOD FOR STOPPING AGING OF THE BODY BY REPLACING THE OLD BIOCATHODE CENTERS BY YOUNG BIOCATHODE CENTERS.

2. BIOCATHODE CENTERS IN THE BODY ARE PLEXUS CHORIOIDEUS, EPIPHYSIS CEREBRY, GLOMUS CAROTICUM, GLOMUS SUPRACARDIALE AND GLOMUS COCCIGEUM.

3. MARTIN DENEV WAS REGISTERING THE DISCOVERY OF THE BIOCATHODE CENTERS 22 OKTOBER 1964 AT THE BULGARIAN MINISTRY OF PUBLIC HEALTH AND SOCIAL CARE UNDER REGISTRATION NUMMBER X 1823 OF 22 OKTOBER 1964.

## Amended claims

### Amended claims in accordance with Rule 137(2) EPC.

**1.** USING OF STEM CELLS TO CREATE YOUNG BIOCATHODE CENTERS, AS PLEXUS CHORIOIDEUS, EPIPHYSIS CEREBRY, GLOMUS CAROTICUM, GLOMUS SUPRACARDIALE AND GLOMUS COCCIGEUM, FOR STOPPING OF AGING OF THE BODY.

**2.** METHOD FOR STOPPING AGING OF THE BODY, BY BREATHING IN, BY LIKE SMOKING CIGAR OR PIPE LIKE DEVICES, WITH NEGATIV AIR OR NEGATIV WATER IONES, WHICH ARE GENERATED BY GENERATOR OF NEGATIV AIR IONS OR GENERATOR OF NEGATIV WATER IONS.

**3.** MEDOD FOR STOPPING AGING OF THE BODY, BY COMPRESSING, DURING THE SKIN, NEGATIV AIR IONS.
